(19) Europäisches Patentamt
European Patent Office
Office européen des brevets

(11) Veröffentlichungsnummer: **0 470 466 A2**

(12) **EUROPÄISCHE PATENTANMELDUNG**

(21) Anmeldenummer: **91112642.3**

(22) Anmeldetag: **27.07.91**

(51) Int. Cl.[5]: **C07D 249/08**, A01N 43/653

(30) Priorität: **09.08.90 DE 4025204**
**10.01.91 DE 4100516**

(43) Veröffentlichungstag der Anmeldung:
**12.02.92 Patentblatt 92/07**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Jautelat, Manfred, Dr.**
**Müllersbaum 28**
**W-5093 Burscheid(DE)**
Erfinder: **Scherkenbeck, Jürgen, Dr.**
**Auf dem Bruch 49**
**W-5090 Leverkusen 3(DE)**
Erfinder: **Stroech, Klaus, Dr.**
**Rolsberger Strasse 22**
**W-5650 Solingen 19(DE)**
Erfinder: **Dutzmann, Stefan, Dr.**
**Kosenberg 10**
**W-4010 Hilden 1(DE)**
Erfinder: **Dehne, Heinz-Wilhelm, Dr.**
**Krischer Strasse 81**
**W-4019 Monheim(DE)**
Erfinder: **Hänssler, Gerd, Dr.**
**Am Arenzberg 58a**
**W-5090 Leverkusen 3(DE)**
Erfinder: **Kuck, Karl-Heinz, Dr.**
**Heerstrasse 24**
**W-4018 Langenfeld(DE)**

(54) **Halogenalkyl-azolyl-Derivate.**

(57) Neue Halogenalkyl-azolyl-Derivate der Formel

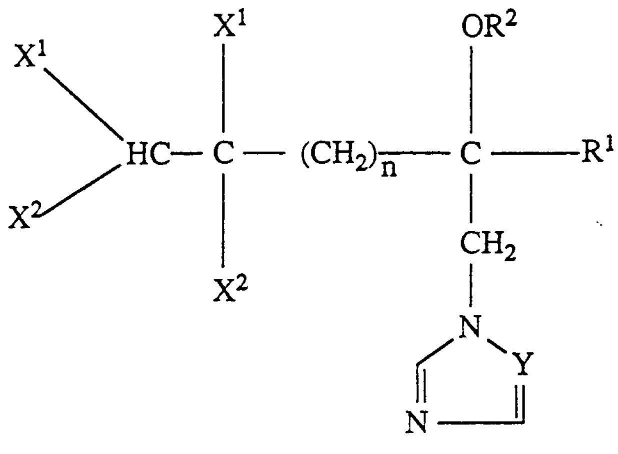

(I),

in welcher
$R^1$ für gegebenenfalls substituiertes Phenyl steht,
$R^2$ für Wasserstoff, Alkyl, Alkenyl, Acyl oder Aralkyl steht,
$X^1$ für Halogen steht,
$X^2$ für Halogen steht,

EP 0 470 466 A2

n für 0 oder 1 steht und
Y für Stickstoff oder eine CH-Gruppe steht,
sowie deren Säureadditions-Salze und Metallsalz-Komplexe, mehrere Verfahren zur Herstellung der neuen Stoffe und deren Verwendung als Mikrobizide im Pflanzenschutz und im Materialschutz.

Die vorliegende Erfindung betrifft neue Halogenalkyl-azolyl-Derivate, mehrere Verfahren zu deren Herstellung und deren Verwendung als Mikrobizide im Pflanzenschutz und im Materialschutz.

Es ist bekannt geworden, daß bestimmte Dihalogen-allyl-triazolyl-Derivate fungizide Eigenschaften besitzen (vgl. EP-OS 0 097 425). So lassen sich z.B. 4-(2,4-Dichlor-phenyl)-1,2-dibrom-4-hydroxy-5-(1,2,4-triazol-1-yl)-pent-1-en und 4-(2,4-Dichlor-phenyl)-1,2-dichlor-4-hydroxy-5-(1,2,4-triazol-1-yl)-pent-1-en zur Bekämpfung von Pilzen einsetzen. Die Wirkung dieser Stoffe ist gut, läßt aber bei niedrigen Aufwandmengen in manchen Fällen zu wünschen übrig.

Weiterhin ist schon bekannt, daß bestimmte Halogenalkyl-triazolyl-Derivate, die am zentralen Kohlenstoffatom einen gegebenenfalls substituierten Phenylrest enthalten, als Fungizide gegen phytopathogene Pilze geeignet sind (vgl. EP-OS 0 380 277). Entsprechende Verbindungen, die einen Halogenalkyl-Rest enthalten, der in Nachbarstellung zum zentralen Kohlenstoffatom zweifach halogeniert ist, werden jedoch nicht beschrieben. Ferner werden auch keine Stoffe dieses Typs offenbart, die am zentralen Kohlenstoffatom einen n-Propyl-Rest aufweisen, der in der 2- und der 3-Position jeweils zweifach halogeniert ist.

Es wurden nun neue Halogenalkyl-azolyl-Derivate der Formel

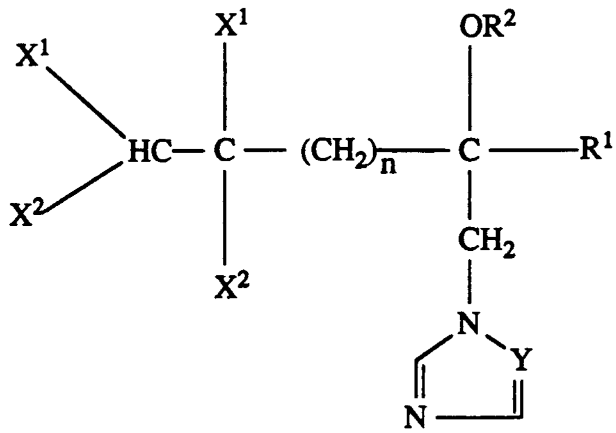

(I),

in welcher

$R^1$ für gegebenenfalls substituiertes Phenyl steht,

$R^2$ für Wasserstoff, Alkyl, Alkenyl, Acyl oder Aralkyl steht,

$X^1$ für Halogen steht,

$X^2$ für Halogen steht,

n für 0 oder 1 steht und

Y für Stickstoff oder eine CH-Gruppe steht,

sowie deren Säureadditions-Salze und Metallsalz-Komplexe gefunden.

Die Verbindungen der Formel (I) enthalten mindestens ein asymmetrisch substituiertes Kohlenstoffatom und können deshalb in optischen Isomerenformen anfallen. Die vorliegende Erfindung betrifft sowohl die Isomerengemische als auch die einzelnen Isomeren.

Weiterhin wurde gefunden, daß man Halogenalkyl-azolyl-Derivate der Formel (I) sowie deren Säureadditions-Salze und Metallsalzkomplexe erhält, wenn man Alkine der Formel

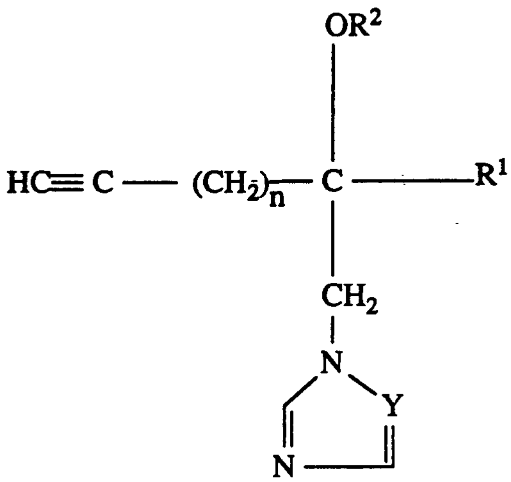

(II),

in welcher
$R^1$, $R^2$, Y und n die oben angegebenen Bedeutungen haben,
mit Halogen oder Halogen liefernden Verbindungen in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen Halogenalkylazolyl-Derivate der Formel (I) sowie deren Säureadditions-Salze und Metallsalz-Komplexe starke mikrobizide Eigenschaften besitzen und sowohl im Pflanzenschutz als auch im Materialschutz eingesetzt werden können.

Überraschenderweise besitzen die erfindungsgemäßen Stoffe sowohl im Pflanzenschutz als auch im Materialschutz eine bessere mikrobizide Wirksamkeit als die konstitutionell ähnlichsten vorbekannten Verbindungen gleicher Wirkungsrichtung. In den Verbindungen der Formeln (I) und (II) steht

$R^1$ vorzugsweise für Phenyl, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, gegebenenfalls einfach oder zweifach, gleichartig oder verschieden, durch Halogen substituiertes Phenyl, gegebenenfalls einfach oder zweifach, gleichartig oder verschieden, durch Halogen substituiertes Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano.

$R^2$ steht vorzugsweise für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil.

$X^1$ steht vorzugsweise für Fluor, Chlor oder Brom.

$X^2$ steht vorzugsweise für Fluor, Chlor oder Brom.

Besonders bevorzugt steht

$R^1$ für Phenyl, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Phenyl, Chlorphenyl, Dichlorphenyl, Phenoxy, Chlorphenoxy, Dichlorphenoxy, Methoxycarbonyl, Ethoxycarbonyl, Methoximinomethyl, 1-Methoximinoethyl, Nitro und/oder Cyano.

$R^2$ steht besonders bevorzugt für Wasserstoff, Methyl, Ethyl, n-Propyl, Isopropyl, n-Butyl, Allyl, Formyl, Acetyl, Benzyl oder Phenethyl.

$X^1$ steht besonders bevorzugt für Fluor, Chlor oder Brom.

$X^2$ steht besonders bevorzugt für Fluor, Chlor oder Brom.

Der Index n steht für 0 oder 1.

Y steht für Stickstoff oder eine CH-Gruppe.

Bevorzugte erfindungsgemäße Stoffe sind auch Additionsprodukte aus Säuren und Halogenalkyl-azolyl-Derivaten der Formel (I), in denen $R^1$, $R^2$, $X^1$, $X^2$, Y und n die Bedeutungen haben, die bereits als bevorzugt oder besonders bevorzugt für diese Reste und den Index n genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifünktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure, Camphersulfonsäure oder Saccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und Halogenalkyl-azolyl-Derivaten der Formel (I), in denen $R^1$, $R^2$, $X^1$, $X^2$, Y und n die Bedeutungen haben, die bereits als bevorzugt oder besonders bevorzugt für diese Reste und den Index n genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Halogenalkyl-azolyl-Derivate genannt.

Tabelle 1

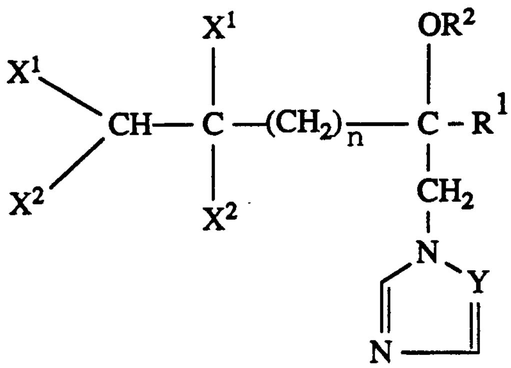

| $X^1$ | $X^2$ | n | $R^1$ | $R^2$ | Y |
|---|---|---|---|---|---|
| Cl | Cl | 0 | 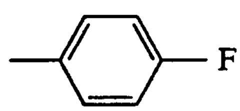 | H | N |
| Cl | Cl | 1 | 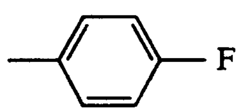 | H | N |
| Cl | Cl | 1 | 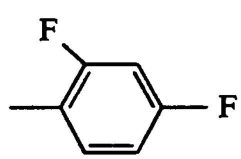 | H | N |
| Cl | Br | 1 | 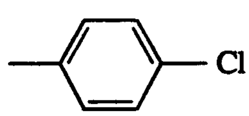 | H | N |

Tabelle 1 (Fortsetzung)

| $X^1$ | $X^2$ | n | $R^1$ | $R^2$ | Y |
|---|---|---|---|---|---|
| Cl | Cl | 0 | 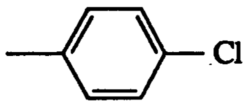 | H | CH |
| Cl | Cl | 0 | 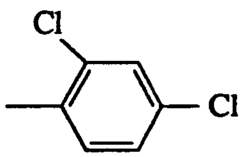 | H | N |
| Cl | Cl | 0 | 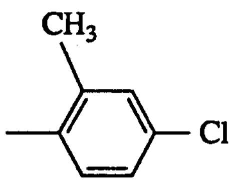 | H | N |

Tabelle 1 (Fortsetzung)

| $X^1$ | $X^2$ | n | $R^1$ | $R^2$ | Y |
|---|---|---|---|---|---|
| Cl | Cl | 0 | 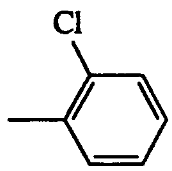 | H | N |
| Cl | Cl | 0 | 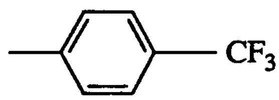 | H | N |
| Cl | Cl | 1 | 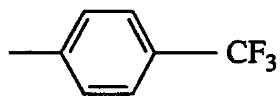 | H | N |
| Cl | Cl | 0 | 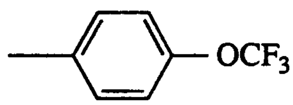 | H | N |
| Cl | Cl | 1 | 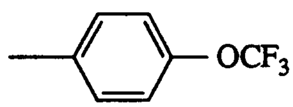 | H | N |
| Cl | Cl | 0 | 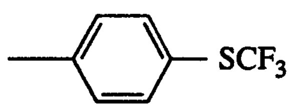 | H | N |
| Cl | Cl | 1 | 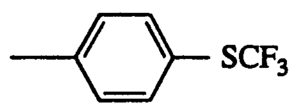 | H | N |

Verwendet man 3-(4-Chlorphenyl)-3-hydroxy-4-(1,2,4-triazol-1-yl)-but-1-in als Ausgangsstoff und Chlorgas als Reaktionskomponente, so kann der Verlauf des erfindungsgemäßen Verfahrens (a) durch das folgende Formelschema veranschaulicht werden:

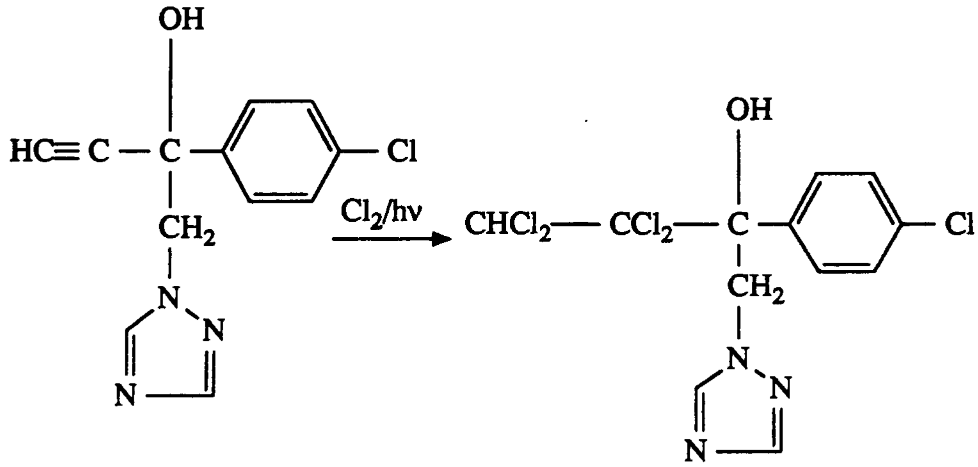


Die bei der Durchführung des erfindungsgemäßen Verfahrens als Ausgangsstoffe benötigten Alkine der Formel (II) sind teilweise bekannt (vgl. EP-OS 0 096 786 und EP-OS 0 353 558). Sie lassen sich herstellen, indem man

a) Azolyl-methyl-ketone der Formel

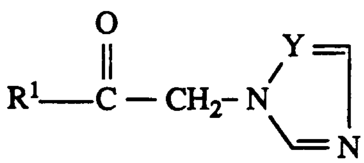 (III),

in welcher

$R^1$ und Y die oben angegebenen Bedeutungen haben,

entweder

α) mit Acetylen-Salzen der Formel

HC≡CMe (IV),

in welcher

Me für ein Äquivalent eines Metallkations steht,

in Gegenwart eines Verdünnungsmittels umsetzt,

oder

β) mit Propargylhalogeniden der Formel

$HC{\equiv}C{-}CH_2{-}Hal$ (V),

in welcher

Hal für Chlor oder Brom steht,

in Gegenwart von aktiviertem Aluminium und in Gegenwart eines Verdünnungsmittels umsetzt, und gegebenenfalls die dabei entstehenden Alline der Formel

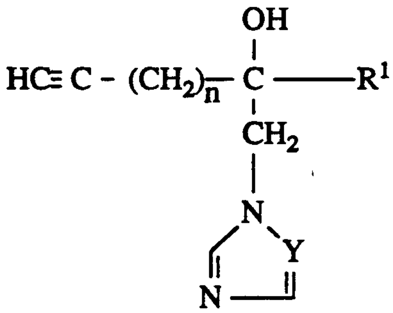

(IIa),

in welcher

$R^1$, Y und n die oben angegebenen Bedeutungen haben,

mit starken Basen in Gegenwart eines Verdünnungsmittels umsetzt und die dabei entstehenden Alkoholate der Formel

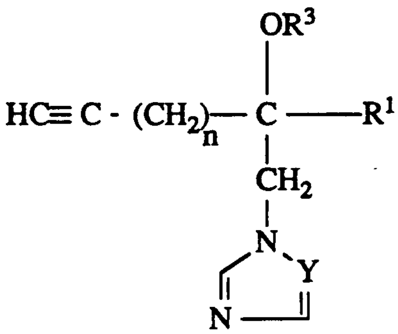

(IIb),

in welcher

$R^1$, Y und n die oben angegebenen Bedeutungen haben und

$R^3$ für einen kationischen Rest einer Base steht,

mit Halogen-Verbindungen der Formel

$R^4$-Hal' (VI),

in welcher

$R^4$ für Alkyl, Alkenyl, Acyl oder Aralkyl seht und

Hal' für Chlor, Brom der Iod steht,

in Gegenwart eines Verdünnungsmittels umsetzt,

oder

b) Chlormethylketone der Formel

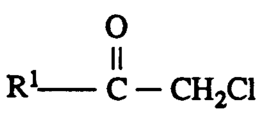

(VII),

in welcher

$R^1$ die oben angegebene Bedeutung hat,

entweder

α) mit Acetylenen der Formel

$HC{\equiv}CR^5$ (VIII),

in welcher

$R^5$ für Wasserstoff oder ein Äquivalent eines Metallkations steht,
gegebenenfalls in Gegenwart einer Base und in Gegenwart eines Verdünnungsmittels umsetzt,
oder
*β*) mit Propargylhalogeniden der Formel

$HC\equiv C-CH_2-Hal$ (V),

in welcher
Hal die oben angegebene Bedeutung hat,
unter den Bedingungen des Verfahrens (a), Variante *β*, umsetzt,
und dann die dabei entstehenden Hydroxyalkine der Formel

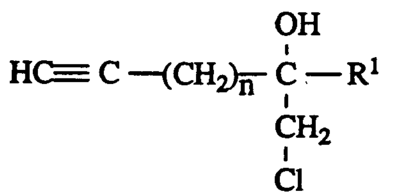

(IX),

in welcher
$R^1$ und n die oben angegebenen Bedeutungen haben,
mit Azolen der Formel

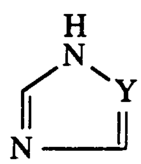

(X),

in welcher
Y die oben angegebene Bedeutung hat,
in Gegenwart eines Säurebindemittels und in Gegenwart eines Verdünnungsmittels umsetzt und gegebenenfalls die dabei entstehenden Alkine der Formel

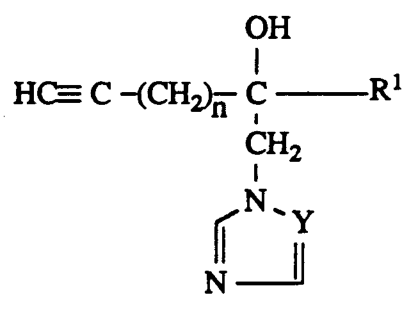

(IIa),

in welcher
$R^1$, Y und n die oben angegebenen Bedeutungen haben,
gemäß Verfahren (a) weiter umsetzt.

Die bei der Durchführung des Verfahrens (a) als Ausgangsstoffe benötigten Azolylmethyl-ketone der Formel (III) sind bekannt oder lassen sich nach prinzipiell bekannten Verfahren in einfacher Weise herstellen (vgl. DE-OS 2 431 407 und EP-OS 0 353 558).

Die bei der Durchführung des Verfahrens (a), Variante α, als Reaktionskomponenten benötigten Acetylen-Salze sind durch die Formel (IV) allgemein definiert. In dieser Formel steht Me vorzugsweise für

ein Lithiumkation oder für ein Äquivalent eines Cer(III)-Kations.

Die Acetylen-Salze der Formel (IV) sind bekannt (vgl. Houben-Weyl, "Methoden der Organischen Chemie", Bd. V/2a, Seiten 509 ff., Georg Thieme Verlag, Stuttgart 1977 und Tetrahedron Letters 25, (1984)-4233).

Als Verdünnungsmittel können bei der Durchführung der ersten Stufe des Verfahrens (a), Variante α, alle für derartige Umsetzungen üblichen inerten organischen Solventien verwendet werden. Vorzugsweise in Frage kommen Ether, wie Tetrahydrofuran oder Diethylether, und außerdem Kohlenwasserstoffe, wie n-Hexan.

Die Reaktionstemperaturen können bei der Durchführung der ersten Stufe des Verfahrens (a), Variante α, innerhalb eines größeren Bereiches variiert werden. Im allgemeinen arbeitet man bei Temperaturen zwischen -78°C und +30°C, vorzugsweise bei Temperaturen zwischen -70°C und +20°C.

Bei der Durchführung des Verfahrens (a) arbeitet man ebenso wie bei der Durchführung des erfindungsgemäßen Verfahrens und des Verfahrens (b) im allgemeinen unter Normaldruck.

Man geht bei der Durchführung der ersten Stufe des Verfahrens (a), Variante α, im allgemeinen so vor, daß man zunächst die Acetylen-Salze herstellt und diese dann ohne vorherige Isolierung mit einer äquivalenten Menge, bzw. einem Über- oder Unterschuß an Azolyl-methyl-keton der Formel (III) umsetzt. Die Aufarbeitung erfolgt nach üblichen Methoden. Im allgemeinen geht man so vor, daß man das Reaktionsgemisch zunächst mit einer wäßrigen Salz-Lösung, z.B. Ammoniumchlorid-Lösung, versetzt, dann mehrfach mit einem in Wasser wenig löslichen organischen Solvens ausschüttelt und die vereinigten organischen Phasen nach dem Trocknen unter vermindertem Druck einengt.

Die bei der Durchführung des Verfahrens (a), Variante β, als Reaktionskomponenten benötigten Propargylhalogenide der Formel (V) sind bekannt.

Als Verdünnungsmittel können bei der Durchführung der ersten Stufe des Verfahrens (a), Variante β, alle für derartige Umsetzungen üblichen inerten organischen Solventien verwendet werden. Vorzugsweise in Frage kommen Ether, wie Tetrahydrofuran oder Diethylether.

Bei der Durchführung der ersten Stufe des Verfahrens (a), Variante β, arbeitet man in Gegenwart von aktiviertem Aluminium. Dieses wird hergestellt, indem man zu Aluminium-Schuppen katalytische Mengen an Quecksilber-(II)-chlorid und Iod zugibt.

Die Reaktionstemperaturen können bei der Durchführung der ersten Stufe des Verfahrens (a), Variante β, innerhalb eines größeren Bereiches variiert werden. Im allgemeinen arbeitet man bei Temperaturen zwischen -80°C und +100°C, vorzugsweise bei Temperaturen zwischen -70°C und +60°C.

Man geht bei der Durchführung der ersten Stufe des Verfahrens (a), Variante β, im allgemeinen so vor, daß man auf 1 Mol an Azolyl-methyl-keton der Formel (III) 1 bis 2 Mol Propargyl-halogenid der Formel (V) und 1 bis 1,5 Mol Aluminium und katalytische Mengen an Quecksilber-(II)-chlorid und Iod einsetzt. Die Isolierung der entstehenden Produkte erfolgt nach üblichen Methoden.

Bei der Durchführung der zweiten Stufe des Verfahrens (a) erfolgt die Überführung der Alkine der Formel (IIa) in die entsprechenden Alkoholate, indem man mit geeigneten starken Basen, wie Alkalimetallamiden oder -hydriden, quarternären Ammonium-hydroxiden oder Phosphonium-hydroxiden in einem inerten Verdünnungsmittel, wie z.B. Dioxan, bei Raumtemperatur umsetzt. Demgemäß steht $R^3$ in den Verbindungen der Formel (IIb) vorzugsweise für ein Alkalimetallkation, wie ein Natrium- oder Kaliumkation, oder für ein quarternäres Ammonium- oder Phosphoniumkation.

Die bei der Durchführung der dritten Stufe des Verfahrens (a) als Reaktionskomponenten benötigten Halogen-Verbindungen sind durch die Formel (VI) allgemein definiert. In dieser Formel steht $R^4$ vorzugsweise für die Bedeutungen, die bereits im Zusammenhang mit der Beschreibung der erfindungsgemäßen Stoffe der Formel (I) für den Substituenten $R^2$ genannt wurden mit Ausnahme der Bedeutung von Wasserstoff. Hal' steht für Chlor, Brom oder Iod.

Die Halogenverbindungen der Formel (VI) sind bekannt oder lassen sich nach im Prinzip bekannten Methoden herstellen.

Als Verdünnungsmittel kommen bei der Durchführung der zweiten und dritten Stufe des Verfahrens (a) inerte organische Lösungsmittel in Frage. Vorzugsweise verwendbar sind Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung der zweiten und dritten Stufe des Verfahrens (a) in einem größeren Bereich variiert werden. Im allgemeinen arbeitet man bei Temperaturen zwischen 0°C und 120°C, vorzugsweise zwischen 20°C und 100°C.

Bei der Durchführung der zweiten Stufe des Verfahrens (a) setzt man zunächst Alkine der Formel (IIa) mit starken Basen zu den entsprechenden Alkoholaten der Formel (IIb) um. In der folgenden dritten Stufe setzt man auf 1 Mol eines Alkoholates der Formel (IIb) vorzugsweise 1 bis 2 Mol Halogenverbindung der

Formel (VI) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird in der zweiten und dritten Stufe des Verfahrens (a) zweckmäßigerweise so verfahren, daß man von einer Hydroxy-Verbindung der Formel (IIa) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (VI) umsetzt, wobei unter Austritt von Alkalimetallhalogenid die Verbindungen der Formel (II) in einem Arbeitsgang erhalten werden.

Nach einer weiteren bevorzugten Ausführungsform werden zweckmäßigerweise die Herstellung der Alkoholate sowie die Umsetzung mit einer Halogenverbindung der Formel (VI) in einem Zweiphasensystem, wie beispielsweise wäßrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die bei der Durchfürung des Verfahrens (b) als Ausgangsstoffe benötigten Chlormethylketone sind durch die Formel (VII) allgemein definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Chlormethylketone der Formel (VII) sind bekannt oder lassen sich nach prinzipiell bekannten Methoden herstellen (vgl. DE-OS 3 049 461).

Die bei dem Verfahren (b), Variante $\alpha$, als Reaktionskomponenten benötigten Acetylene sind durch die Formel (VIII) allgemein definiert. In dieser Formel steht $R^5$ vorzugsweise für Wasserstoff, ein Lithium-kation oder ein Äquivalent eines Magnesium- oder Cer (III)-Kations.

Die Acetylene der Formel (VIII) sind bekannt.

Als Basen kommt bei der Durchführung der ersten Stufe des Verfahrens (b), Variante $\alpha$, alle für derartige Umsetzungen üblichen starken Basen in Betracht. Vorzugsweise verwendbar sind Alkalimetallhydroxide, wie Kaliumhydroxid.

Als Verdünnungsmittel können bei der Durchführung der ersten Stufe des Verfahrens (b), Variante $\alpha$, alle für derartige Umsetzungen üblichen inerten organischen Solventien verwendet werden. Vorzugsweise in Frage kommen, Ether, wie Tetrahydrofuran oder Diethylether.

Die Reaktionstemperaturen können bei der Durchführung der ersten Stufe des Verfahrens (b), Variante $\alpha$, innerhalb eines größeren Bereichs variiert werden. Im allgemeinen arbeitet man bei Temperaturen zwischen -78 °C und +50 °C, vorzugsweise zwischen -78 °C und +40 °C.

Bei der Durchführung der ersten Stufe des Verfahrens (b), Variante $\alpha$, geht man im allgemeinen so vor, daß man Chlormethylketone der Formel (VII) und Acetylene der Formel (VIII) in angenähert äquivalenten Mengen umsetzt. Es ist jedoch auch möglich, die eine oder andere Komponente in einem Überschuß zu verwenden. Die Aufarbeitung erfolgt nach üblichen Methoden.

Die Durchführung der ersten Stufe des Verfahrens (b), Variante $\beta$, erfolgt unter den Bedingungen, die auch bei der Durchführung der ersten Stufe des Verfahrens (a), Variante $\beta$, angewandt werden.

Die Hydroxyalkine der Formel (IX) können direkt weiter umgesetzt werden mit Azolen der Formel (X). Sie können aber auch zunächst in Oxirane überführt werden und dann mit Azolen der Formel (X) umgesetzt werden.

Bei der Durchführung der zweiten Stufe des Verfahrens (b) kommen als Säurebindemittel alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diaza-bicyclo[2.2.2]octan (DABCO).

Als Verdünnungsmittel kommen bei der Durchführung der zweiten Stufe des Verfahrens (b) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, sowie tert.-Butylmethylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (b)

innerhalb eines größeren Bereiches variiert werden. Im allgemeinen arbeitet man bei Temperaturen zwischen 0°C und 200°C, vorzugsweise zwischen 20°C und 150°C.

Bei der Durchführung der zweiten Stufe des Verfahrens (b) geht man im allgemeinen so vor, daß man auf 1 Mol an Hydroxyalkin der Formel (IX) eine äquivalente Menge oder auch einen Überschuß an Azol der Formel (X) sowie 2 bis 3 Mol an Säurebindemittel einsetzt. Die Aufarbeitung erfolgt nach üblichen Methoden. Die gegebenenfalls gewünschte weitere Umsetzung der Alkine der Formel (IIa) erfolgt bei dem Verfahren (b) in gleicher Weise wie bei dem Verfahren (a).

Als Halogene kommen bei der Durchführung des erfindungsgemäßen Verfahrens vorzugsweise Fluor, Chlor und Brom als Reaktionskomponenten in Betracht, ferner gemischte Halogene wie Chlor(I)-fluorid, Brom(I)-fluorid oder Brom(I)-chlorid, (s. Methodicum Chimicum, F.Korte, Bd. 7, S. 842 (1976)).

Als Halogen liefernde Verbindungen können beispielsweise Sulfurylchlorid, N-Bromsuccinimid mit Salzsäure, N-Chlorsuccinimid mit Bromwasserstoffsäure oder N-Chlorsuccinimid mit Fluorwasserstoff/Pyridin (s. Synthesis 1973, 780) verwendet werden.

Die Addition der Halogene an die Alkine der Formel (II) kann durch Einwirkung von Licht, durch Wärme, durch radikalbildende Substanzen, wie organische Peroxide, durch oberflächenaktive Stoffe, wie Aktivkohle, oder Metallsalze,wie Kupfer(II)-chlorid oder Eisen(III)-chlorid, begünstigt werden.

Als Verdünnungsmittel können bei der Durchführung des erfindungsgemäßen Verfahrens alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung des erfindungsgemäßen Verfahrens setzt man auf 1 Mol an Alkin der Formel (II) im allgemeinen eine äquivalente Menge oder einen Überschuß an Halogen bzw. Halogen liefernder Verbindung ein. Wenn es beabsichtigt ist, Halogenalkyl-azolyl-Derivate der Formel (I) herzustellen, in denen $X^1$ und $X^2$ verschieden sind, so geht man zweckmäßigerweise so vor, daß man zunächst 1 Mol eines bestimmten Halogens an das Alkin der Formel (II) addiert und danach in einem zweiten Schritt 1 Mol eines anderen Halogens addiert. Die Aufarbeitung erfolgt nach üblichen Methoden. Im allgemeinen geht man so vor, daß man mit einem in Wasser wenig löslichen organischen Solvens verdünnt, mit Wasser wäscht und die organische Phase nach dem Trocknen einengt. Es ist jedoch auch möglich, das Reaktionsgemisch nach beendeter Umsetzung direkt durch Abziehen der flüchtigen Komponenten unter vermindertem Druck einzuengen. Die entstehenden Produkte können gegebenenfalls nach üblichen Methoden weiter gereinigt werden.

Die nach dem erfindungsgemäßen Verfahren erhältlichen Halogenalkyl-azolyl-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Kondienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botryrtis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella, Erysiphe, Fusarium, Pyrenophora, Cochliobolus, Septoria, Pyricularia und Pellicularia, sowie zur Bekämpfung von Gurkenmehltau und Apfelschorf und außerdem zur Bekämpfung von Botrytis im Obst-, Wein- und Gemüsebau. Sie besitzen außerdem eine gute und breite in - vitro - Wirkung und eignen sich zur Bekämpfung echter Mehltaupilze, wie Rhizoctonia solani.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,

Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa
Staphylococcus, wie Staphylococcus aureus.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol,Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können bei Verwendung im Pflanzenschutz als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt:
Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihydroxy-5,5'-dichlor)-diphenylmethan und 3-Methyl-4-chlor-phenol, Organo-Zinnverbindungen, Trihalogenmethylthio-Verbindungen wie Folpet, Fluorfolpet, Dichlofluanid.

Die Herstellung und die Verwendung der erfindungsgemäßen Wirkstoffe werden durch die folgenden Beispiele veranschaulicht.

Herstellungsbeispiele

Beispiel 1

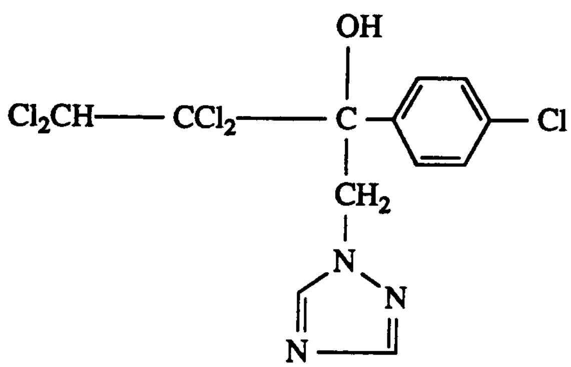

(I-1)

2,5 g(10 mMol) 3-(4-Chlorphenyl)-3-hydroxy-4-(1,2,4-triazol-1-yl)-but-1-in werden in 50 ml Methylenchlorid bei 10 °C unter Belichtung mit einer 300 Watt UV-Lampe durch Einleiten von Chlorgas innerhalb von 30 Stunden chloriert. Die dabei entstehende Suspension wird zur Aufarbeitung mit Methylenchlorid verdünnt und mit wäßriger Natriumcarbonat-Lösung ausgeschüttelt. Die organische Phase wird nach dem Trocknen über Natriumsulfat unter vermindertem Druck eingeengt. Man erhält auf diese Weise 3,8 g (97 % der Theorie) an 3-(4-Chlorphenyl)-3-hydroxy-4-(1,2,4-triazol-1-yl)-1,1,2,2-tetrachlorbutan in Form einer Festsubstanz vom Schmelzpunkt 168 bis 169 °C.

$^1$H-NMR (200 MHz; DMSO): $\delta$ = 5,05 und 5,45 (AB-System; 2H), 6,4(s; 1H), 7,1(OH), 7,35 und 7,7 (4H), 7,75(s; 1H), 8,35(s;1H).

Herstellung der Ausgangssubstanz:

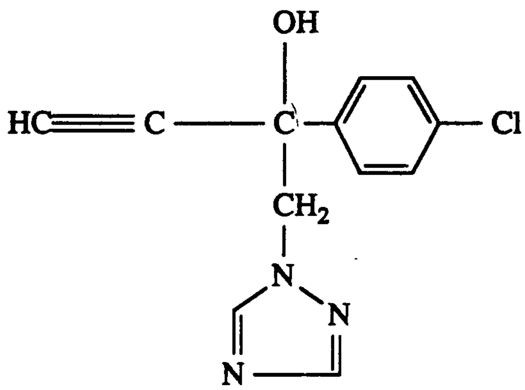

(II-1)

5,0 g (23,2 mMol) 4-Chlor-3-(4-chlorphenyl)-3-hydroxy-1-butin werden in 50 ml abs. Acetonitril mit 1,6 g (23,3 mMol) 1,2,4-Triazol und 6,4 g (46,4 mMol) wasserfreiem Kaliumcarbonat 5,5 Stunden unter Rückfluß erhitzt. Dann verdünnt man mit Dichlormethan und schüttelt mehrfach mit Wasser aus. Nach dem Trocknen und Einengen der organischen Phase erhält man 5,8 g Rohprodukt, das nach Filtration mit Essigsäureethylester über 100 g Kieselgel und erneutem Einengen kristallisiert. Man erhält auf diese Weise 3-(4-Chlorphenyl)-3-hydroxy-4-(1,2,4-triazolyl-1)-1-butin in Form einer Festsubstanz vom Schmelzpunkt 90-94° C.

$^{1}$H-NMR ($CDCl_3$): δ 2,65 (s, 1H), 4,4 (AB, 2H), 5,8 (OH), 7,35 und 7,45 (4H), 7,85 (s, 1H), 8,15 (s, 1H).

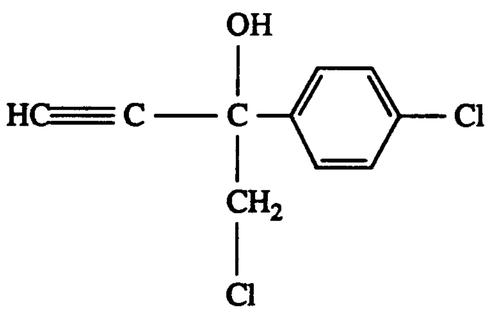

(IX-1)

Zu der Lösung von 5,2 g (0,2 Mol) Acetylen in 200 ml absolutem Tetrahydrofuran bei 70° C werden 80 ml (0,2 Mol) 23 % Butyllithiumlösung in Hexan zugetropft. Nach 10 Minuten wird die Lösung von 28,35 g (0,15 Mol) 4,ω-Dichloracetophenon in 100 ml absolutem Tetrahydrofuran eingetragen. Es wird 2 Stunden bei -70° C nachgerührt, und durch Zutropfen des Gemischs aus 20 ml Methanol und 20 ml Essigsäure abgestoppt. Das Lösungsmittel wird im Vakuum abgezogen und der Rest nach Lösen in Dichlormethan mit gesättigter, wäßriger Ammoniumchloridlösung mehrfach ausgeschüttelt. Nach Trocknen und Abdestillieren der organischen Phase erhält man 27,8 g Rohprodukt mit einem Gehalt von 76,3 % 4-Chlor-3-(4-chlorphenyl)-3-hydroxy-1-butin und 17,6 % Ausgangsmaterial, das nach Kristallisation abgesaugt wird.

$^{1}$H-NMR ($CDCl_3$): δ = (2,8(s, 1H) ,3,4(OH) ,375 (AB, 2H),7,4 und 7,65(4H).

Nach der im Beispiel 1 angegebenen Methode werden auch die in der folgenden Tabelle formelmäßig aufgeführten Stoffe hergestellt:

<u>Tabelle 2</u>

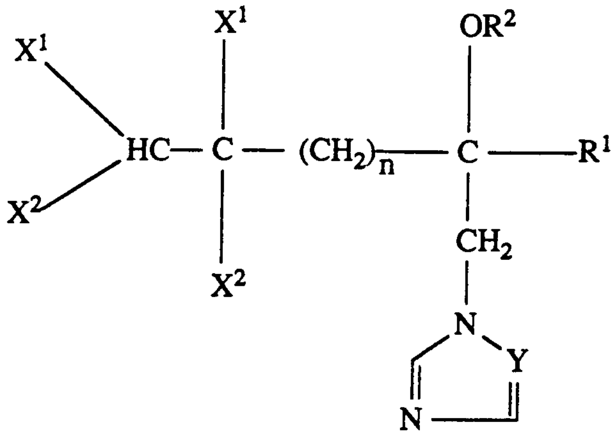 (I)

| Beispiel Nr. | Verb. Nr. | $X^1$ | $X^2$ | n | $R^1$ | $R^2$ | Y | Physikalische Konstante |
|---|---|---|---|---|---|---|---|---|
| 2 | I-2 | Cl | Cl | 1 | 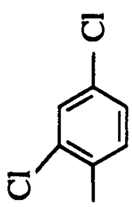 | H | N | GC/MS(CI):436(M+H$^+$) |
| 3 | I-3 | Cl | Cl | 1 | 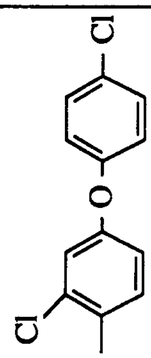 | H | N | Schmelzp. 142-144°C |
| 4 | I-4 | Cl | Cl | 1 | 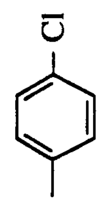 | H | N | NMR (200 MHz, $CDCl_3$): δ 3,15 (AB,2H), 4,5 (AB,2H), 7,6 (s,1H), 7,3 (4H), 7,9 (s,1H), 8,0 (s,1H) |
| 5 | I-5 | Cl | Cl | 1 | 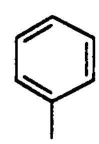 | H | N | GC/MS (CI): 368 (M+H$^+$) |

In den folgenden Verwendungsbeispielen wurden die Verbindungen der nachstehend aufgeführten Formeln als Vergleichssubstanzen eingesetzt:

(A) = 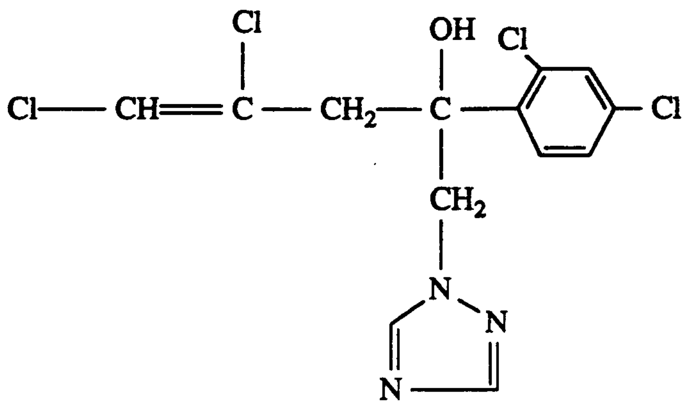

(Bekannt aus EP-OS 0 097 425).

(B) = 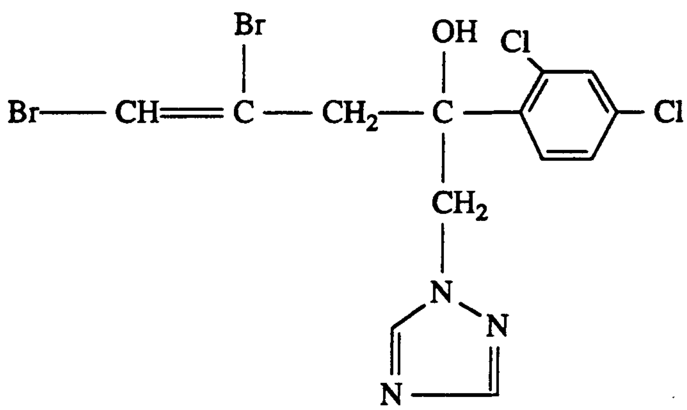

(Bekannt aus EP-OS 0 097 425).

Beispiel A

Erysiphe-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca 80 % aufgestellt, um die Entwicklung von Mehltaupusteln zu begünstigen.

7 Tage nach der Inokulation erfolgt die Auswertung.

In diesem Test zeigen die erfindungsgemäßen Stoffe (I-1), (I-2), (I-4) und (I-5) eine wesentlich bessere Wirksamkeit als die Vergleichssubstanzen (A) und (B).

Beispiel B

Erysiphe-Test (Weizen)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (I-1), (I-2), (I-4) und (I-5) eine wesentlich bessere Wirksamkeit als die Vergleichssubstanzen (A) und (B).

**Patentansprüche**

**1.** Halogenalkyl-azolyl-Derivate der Formel

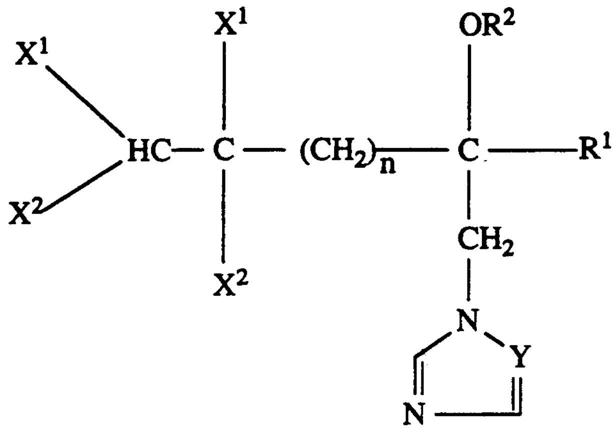

(I),

in welcher

$R^1$ für gegebenenfalls substituiertes Phenyl steht,

$R^2$ für Wasserstoff, Alkyl, Alkenyl, Acyl oder Aralkyl steht,

$X^1$ für Halogen steht,

$X^2$ für Halogen steht,

n für 0 oder 1 steht und

Y für Stickstoff oder eine CH-Gruppe steht,

sowie deren Säureadditions-Salze und Metallsalz-Komplexe.

**2.** Halogenalkyl-azolyl-Derivate der Formel (I) gemäß Anspruch 1,

in denen

$R^1$ für Phenyl steht, das einfach bis dreifach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, gegebenenfalls einfach oder zweifach, gleichartig oder verschieden durch Halogen substituiertes Phenyl, gegebenenfalls einfach oder zweifach, gleichartig oder verschieden durch Halogen substituiertes Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,

$R^2$ für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,

$X^1$ für Fluor, Chlor oder Brom steht,
$X^2$ für Fluor, Chlor oder Brom steht,
n für 0 oder 1 steht und
Y für Stickstoff oder eine CH-Gruppe steht.

**3.** Verfahren zur Herstellung von Halogenalkyl-azolyl-Derivaten der Formel

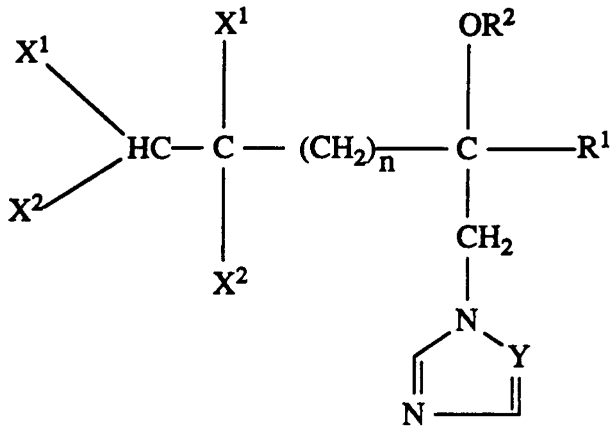

(I),

in welcher
$R^1$ für gegebenenfalls substituiertes Phenyl steht,
$R^2$ für Wasserstoff, Alkyl, Alkenyl, Acyl oder Aralkyl steht,
$X^1$ für Halogen steht,
$X^2$ für Halogen steht,
n für 0 oder 1 steht und
Y für Stickstoff oder eine CH-Gruppe steht,
sowie von deren Säureadditions-Salzen und Metallsalz-Komplexen,

dadurch gekennzeichnet, daß man
Alkine der Formel

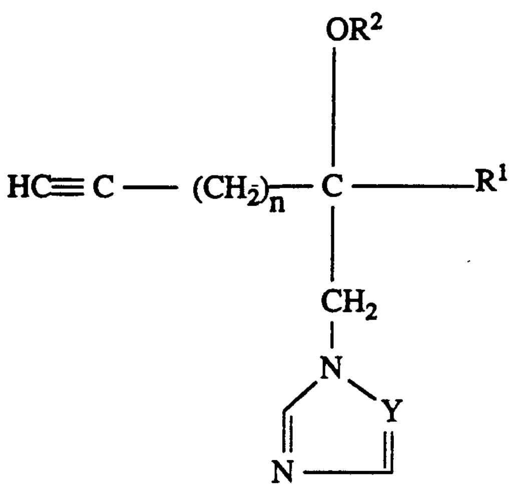

(II),

in welcher
$R^1$, $R^2$, Y und n die oben angegebenen Bedeutungen haben,
mit Halogen oder Halogen liefernden Verbindungen in Gegenwart eines Verdünnungsmittels umsetzt,

und gegebenenfalls anschließend an die so erhaltenen Verbindungen der Formel (I) eine Säure oder ein Metallsalz addiert.

**4.** Mikrobizide Mittel, gekennzeichnet durch einen Gehalt an mindestens einem Halogenalkyl-azolyl-Derivat der Formel (I) gemäß Anspruch 1 bzw. an einem Säureadditions-Salz oder Metallsalz-Komplex eines Halogenalkyl-azolyl-Derivates der Formel (I).

**5.** Verwendung von Halogenalkyl-azolyl-Derivaten der Formel (I) gemäß Anspruch 1 bzw. von deren Säureadditions-Salzen und Metallsalz-Komplexen als Mikrobizide im Pflanzenschutz und im Materialschutz.

**6.** Verfahren zur Bekämpfung von unerwünschten Mikroorganismen im Pflanzenschutz und im Materialschutz, dadurch gekennzeichnet, daß man Halogenalkyl-azolyl-Derivate der Formel (I) gemäß Anspruch 1 bzw. deren Säureadditions-Salze oder Metallsalz-Komplexe auf die Mikroorganismen und/oder ihren Lebensraum ausbringt.

**7.** Verfahren zur Herstellung von mikrobiziden Mitteln, dadurch gekennzeichnet, daß man Halogenalkyl-azolyl-Derivate der Formel (I) gemäß Anspruch 1 bzw. deren Säureadditionssalze oder Metallsalz-Komplexe mit Streckmitteln und/oder oberflächenaktiven Stoffen vermischt.